# EUROPEAN PATENT SPECIFICATION

(11) **EP 1 475 096 B1**
(45) Date of publication and mention of the grant of the patent: **11.10.2006**
(21) Application number: 04075998.7
(22) Date of filing: 05.04.2004
(51) Int. Cl.: A61K 33/08, A61K 33/10, A61P 31/22

(54) **Pharmaceutical composition for the treatment of herpes zoster**
Pharmazeutische Zusammensetzungen zur Behandlung von Herpes Zoster
Compositions pharmaceutiques pour le traitement de l'herpès-zoster

(30) Priority: 08.04.2003 ES 200300828
(43) Date of publication of application: 10.11.2004
(73) Proprietor: Nubia Perez de Cabrales, Mercedes, Heredia (CR)
(72) Inventor: Nubia Perez de Cabrales, Mercedes, Heredia (CR)
(74) Representative: Davila Baz, Angel

(56) References cited:
- US-A- 5 476 650
- BILTZ, KLEMM, FISCHER: "Experimentelle Einführung in die Anorganische Chemie" 1 January 1985 (1985-01-01), DE GRUYTER , NEW YORK , XP002288173 * page 96 *
- HOLLEMANN A F, WIBERG E: "Lehrbuch derAnorganischen Chemie" 1 January 1985 (1985-01-01), WALTER DE GRUYTER , NEW YORK , XP002288174 * page 919 *

## Description

### Field of the Invention

The present invention refers to a pharmaceutical composition of topical application, based on calcium carbide ash and vaseline, useful for the treatment of Herpes Zoster.

### Background of the Invention

In recent times, cutaneous diseases have considerably increased among people. The majority of such diseases is caused by viruses. In many of them, the viruses can remain in the human organism in a period of latency, without damaging the organism.

Herpes Zoster is one of the skin diseases caused by the reactivation of the varicella-zoster virus (VZV). Said reactivation occurs most frequently in the elderly or in immunodepressed people. VZV is part of the herpes virus family and is the etiological agent of childhood varicella, after which the virus remains in a latent state in the adjoining dorsal root ganglia. Many years later, as elderly adults and in conditions of immunodepression, emotional stress, neoplasia, etc., the virus is reactivated and causes vesicular lesions on the skin, preceded by pruritus, pain or a sensation of hyperesthesia, which form erythematous patches. These lesions are primarily located in the cephalic, cervical and lumber regions. During the infection of the host cells, the varicella-zoster virus has the shortest replication cycle of all the human herpes viruses. Among the most frequent complications, post-herpetic neuralgia, bacterial overinfection of the ocular type, which can be chronic and cause ophthalmic nerve disorders, and visceral disseminations in immunodepressed patients, stand out.

A feature of viruses is that they are not susceptible to being eliminated by means of treatment with antibiotics. As a result, chemical products acting on the viral replication, inhibiting it, are used. However, there is still no composition that is 100% effective as antiviral treatment.

In the state of the art, there are drugs, either oral administration drugs or topical application drugs, which have shown a very good efficacy in the treatment of viral diseases caused by the Herpes Zoster virus. Among said drugs are acyclovir (acycloguanosine), famciclovir, ribavirin, ganciclovir, sorivudine, etc. Treatments with steroids, interferons, etc., have also been developed. Acyclovir was the first antiviral drug of systemic application which needs to be triphosphorylated in order to exercise its antiviral activity, its absorption being from 10 to 20%. Treatment with said pharmaceutical product requires from 15 to 30 days of administration. However, it has been observed that the required dose of many of these products mentioned is such that it causes counterproductive and unsatisfactory effects: toxicity problems in vital organs such as the liver and kidneys (reversible nephropathies), gastrointestinal disorders and disorders in neuronal cells.

The previously mentioned toxicity problems are a drawback for health and, therefore, alternative solutions are sought by means of the use of other antiviral pharmaceutical products which do not damage the human organism and act with high efficacy against viruses.

Another problem of such antiviral pharmaceutical products is the high price at which they are marketed due to the high costs of their research and of their large-scale manufacture.

Therefore, there is a need to provide an effective composition for the treatment of Herpes Zoster which prevents the toxicity problems presented by the known compositions, and which at the same time has a cost-effective preparation.

US 5 476 650 A (Patel Manilal) discloses a hair relaxing composition comprising inter alias mineral oil, water, petrolatum, calcium hydroxide, a cationic polymer and alcohol, which is used in a high pH hair relaxer system. On the other hand, Biltz et al. ("Experimentelle Einführung in die Anorganische Chemie" 1 January 1985) and Hollemann A F et al. ("Lehrbuch der Anorganischen Chemie" 1 January 1985) describe the reaction of calcium carbide and water for obtaining acetylene and calcium hydroxide. Hollemann also cites different destinations of the calcium carbide production.

### Description of the Invention

It has surprisingly been found that a pharmaceutical composition based on calcium carbide ash and vaseline, topically applied, is useful in the treatment of Herpes Zoster.

According to a first aspect, the present invention refers to a process for obtaining said pharmaceutical composition which comprises the step of obtaining calcium carbide ash from calcium carbide stones, either at ambient humidity or by wetting the stones, characterized in that it also comprises the steps of:
a) drying the obtained calcium carbide ash;
b) sieving the calcium carbide ash;
c) mixing the calcium carbide ash with vaseline at an ash to vaseline ratio of between 1:5 and 1:10 by weight until reaching homogeneity.

According to a further object, the present invention refers to the use of calcium carbide ash in the preparation of a pharmaceutical composition of topical application for the treatment of Herpes Zoster.

The present invention for preparing the pharmaceutical composition uses calcium carbide in the form of stones to obtain calcium carbide ash as the active ingredient. Said ash is combined with a vehicle element, vaseline, for the purpose of producing a composition in the form of a homogenous paste with pharmaceutical aims. Vaseline is a pharmaceutically accepted vehicle.

To obtain said calcium carbide ash, the calcium carbide stones are wetted directly or are exposed to ambient humidity. If they are wetted, the reaction will be faster than if they are exposed to a humid environment. Then, the ash obtained from the reaction between the water and the calcium carbide is dried and then sieved to eliminate unwanted elements, such as calcium carbide stones which have not reacted. The sieved ash is subsequently mixed with vaseline, stirring the mixture at room temperature until obtaining a gray-colored homogenous paste.

According to a preferred embodiment, in order to obtain a finer particle size, once the ash has been sieved, it can be subjected to a pulverization step.

According to another preferred embodiment, between 1 and 5% by weight, with regard to the total weight of the composition, of essential oils from aromatic plants can optionally be added to said calcium carbide ash and vaseline paste for the purpose of aromatizing the composition and making it pleasant to smell.

### Preferred Embodiment of the Invention

To better explain the invention, several preferred embodiment examples are described below with a descriptive and never limiting character.

### Example 1: Preparation of a composition according to the invention with 9% of calcium carbide ash.

Calcium carbide stones, to which a small amount of water has been added, are placed in a previously sterilized recipient, the carbide ash being thus obtained. Then, said ash is passed through a sieve to obtain a fine ash free of unwanted elements. Said ash is subsequently dried and 9.9 g of sieved and dry ash are weighed. Said amount of ash is subsequently mixed with 100 g of vaseline, stirring at room temperature. The mixing of the ash and vaseline ends when a gray-colored homogenous paste is obtained. Optionally, between 1 and 5% by weight of essential oils from aromatic plants are added for the purpose of providing the obtained paste with a pleasant odor.

A fine film of this mixture was applied directly on the regions of the skin affected by the Herpes Zoster virus once a day, and left uncovered. The effects of the virus disappeared after five days.

### Example 2: Preparation of a composition according to the invention with 13% of calcium carbide ash.

In this embodiment, the calcium carbide stones are exposed to the ambient humidity to obtain calcium carbide ash. The ash is subsequently dried and 15 g of sieved carbide ash are weighed. Then, said ash is mixed with 100 g of vaseline, stirring the mixture at room temperature, to obtain a gray-colored homogenous paste. Optionally, between 1 and 5% by weight of essential oils are added.

A fine film of this mixture was applied directly on the regions of the skin affected by the Herpes Zoster virus once a day, and left uncovered. The effects of the virus disappeared after five days.

### Example 3: Preparation of a composition according to the invention with 16.7% of calcium carbide ash.

To obtain the calcium carbide ash, proceed according to Example 1. The ash is subsequently dried and 20 g of sieved carbide ash are weighed. Then, the ash is mixed with 100 g of vaseline, stirring the mixture at room temperature until obtaining a gray-colored homogenous paste. Then, between 1 and 5% by weight, with regard to the total weight of the composition, of essential oils can be added to said homogenous paste.

A fine film of this mixture was applied directly on the regions of the skin affected by the Herpes Zoster virus once a day, and left uncovered. The effects disappeared after three days.

The embodiment examples showed that with a higher concentration of calcium carbide ash in the composition, according to the invention, the effects of the Herpes Zoster virus disappeared more quickly.

## Claims

1. A process for obtaining a pharmaceutical composition of topical application for the treatment of Herpes Zoster which comprises the step of obtaining calcium carbide ash from calcium carbide stones, either at ambient humidity or by wetting the stones, **characterized in that** it also comprises the steps of:
a) drying the obtained calcium carbide ash;
b) sieving the calcium carbide ash;
c) mixing the calcium carbide ash with vaseline at an ash to vaseline ratio between 1:5 and 1:10 by weight until reaching homogeneity.

2. A process according to claim 1, **characterized in that** after step c), it comprises a step for mixing between 1 and 5% by weight, with regard to the total weight of the composition, of essential oils from aromatic plants.

3. A process according to claim 3, **characterized in that** after step b), it comprises a step for pulverizing the calcium carbide ash.

4. Use of calcium carbide ash in the preparation of a pharmaceutical composition of topical application for the treatment of Herpes Zoster.

## Patentansprüche

1. Verfahren zur Erhaltung einer topisch zu verabreichenden pharmazeutischen Zusammensetzung für die Behandlung von Herpes Zoster, das den Arbeitsschritt der Carbidkalkdarstellung aus Calciumcarbidbrocken, entweder kraft der Umgebungsfeuchtigkeit oder durch Befeuchtung der Brocken einschließt, **dadurch gekennzeichnet, dass** es ebenfalls folgende Arbeitsschritte umfasst:
a) Trocknung des erhaltenen Carbidkalks;
b) Durchsieben des Carbidkalks;
c) Mischen des Carbidkalks mit Vaseline im Gewichtsverhältnis Carbidkalk zu Vaseline zwischen 1:5 und 1:10 bis zur Erhaltung eines homogenen Gemisches.

2. Verfahren gemäß Anspruch 1, **dadurch gekennzeichnet dass** es einen auf Schritt c) folgenden Schritt umfasst, in dem ätherische Öle von aromatischen Pflanzen in einer Menge von 1 bis 5 Gew.-%, bezogen auf die Gesamtmasse der Zusammensetzung, beigemischt werden.

3. Verfahren gemäß Anspruch 3, **dadurch gekennzeichnet dass** es einen auf Schritt b) folgenden Schritt umfasst, in dem der Carbidkalk zerstäubt wird.

4. Verwendung von Carbidkalk zur Herstellung einer topisch zu verabreichenden pharmazeutischen Zusammensetzung für die Behandlung von Herpes Zoster.

## Revendications

1. Procédé visant à obtenir une préparation pharmaceutique pour application topique destinée au traitement du zona, qui comprend une étape consistant à obtenir des cendres de carbure de calcium à partir de pierres de carbure de calcium, soit à humidité ambiante soit en humidifiant les pierres, **caractérisé en ce qu'**il comprend aussi les étapes consistant à :
a) sécher les cendres de carbure de calcium obtenues ;
b) tamiser les cendres de carbure de calcium ;
c) mélanger les cendres de carbure de calcium à de la vaseline dans une proportion en poids cendre pour vaseline comprise entre 1/5 et 1/10, jusqu'à ce que la préparation soit homogène.

2. Procédé selon la revendication 1, **caractérisé en ce qu'**il comprend après l'étape c) une étape consistant à mélanger entre 1 et 5 % en poids, par rapport au poids total de la composition, d'huiles essentielles de plantes aromatiques.

3. Procédé selon la revendication 2, **caractérisé en ce qu'**il comprend après l'étape b) une étape consistant à pulvériser les cendres de carbure de calcium.

4. Utilisation de cendres de carbure de calcium dans la préparation d'une composition pharmaceutique pour application topique destinée au traitement du zona.
